# EUROPÄISCHE PATENTSCHRIFT

(11) **EP 0 553 174 B1**
(45) Veröffentlichungstag und Bekanntmachung des Hinweises auf die Patenterteilung: **08.07.1998**
(21) Anmeldenummer: 91918141.2
(22) Anmeldetag: 12.10.1991
(51) Int. Cl.: C07D 237/14, A61K 31/50

(54) **ARYLPYRIDAZINONE**
ARYLPYRIDAZINONES
ARYLPYRIDAZINONES

(30) Priorität: 16.10.1990 CH 3306/90
(43) Veröffentlichungstag der Anmeldung: 04.08.1993
(73) Patentinhaber: BYK GULDEN LOMBERG CHEMISCHE FABRIK GMBH, D-78403 Konstanz (DE)
(72) Erfinder: AMSCHLER, Hermann, D-7760 Radolfzell (DE); FIGALA, Volker, D-7753 Allensbach 4 (DE); FLOCKERZI, Dieter, D-7753 Allensbach (DE); ULRICH, Wolf-Rüdiger, D-7750 Konstanz (DE); BEUME, Rolf, D-7750 Konstanz 18 (DE); HÄFNER, Dietrich, D-7750 Konstanz (DE); HANAUER, Guido, D-7752 Reichenau (DE); ELTZE, Manfrid, D-7750 Konstanz (DE); SCHUDT, Christian, D-7750 Konstanz (DE); KILIAN, Ulrich, D-7752 Reichenau 2 (DE)
(86) Internationale Anmeldenummer: EP9101942
(87) Internationale Veröffentlichungsnummer: WO9206963

(56) Entgegenhaltungen:
- EP-A- 0 125 636
- EP-A- 0 163 965

## Beschreibung

### Technisches Gebiet

Die Erfindung betrifft 6-Aryl-3[2H]pyridazinone, ihre Herstellung, Verwendung und sie enthaltende Arzneimittel.

### Stand der Technik

Aus den europäischen Patenten 125 636 und 163 965 sind bestimmte Pyridazinone bekannt, die eine bronchospasmolytische und/oder cardiotonische Wirkung besitzen.

### Beschreibung der Erfindung

Es wurde gefunden, daß die nachstehend beschriebenen 6-Aryl-3[2H]pyridazinone besonders vorteilhafte pharmakologische Wirkungen aufweisen, durch die sie sich von den Verbindungen der europäischen Patente 125 636 und 163 965 in überraschender Weise unterscheiden.

Gegenstand der Erfindung sind 6-Aryl-3[2H]pyridazinone der allgemeinen Formel I 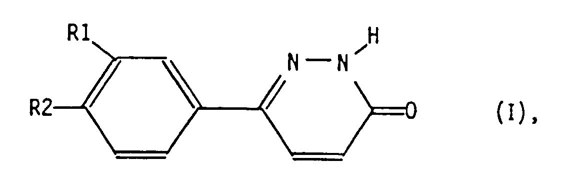 worin einer der Substituenten R1 und R2 Methoxy, Difluormethoxy oder Ethoxy, und der andere C4-C7-Cycloalkoxy oder C3-C7-Cycloalkylmethoxy bedeutet, und ihre Salze mit Basen.

C4-C7-Cycloalkoxy steht beispielsweise für Cyclobutyloxy, Cyclopentyloxy, Cyclohexyloxy und Cycloheptyloxy, wovon Cyclopentyloxy bevorzugt ist.

C3-C7-Cycloalkylmethoxy steht beispielsweise für Cyclopropylmethoxy, Cyclobutylmethoxy, Cyclopentylmethoxy, Cyclohexylmethoxy und Cycloheptylmethoxy, wovon Cyclopropylmethoxy und Cyclobutylmethoxy bevorzugt sind.

Als Salze kommen Salze mit anorganischen und organischen Basen in Betracht. Besonders erwähnt seien die pharmakologisch verträglichen Salze der in der Galenik üblicherweise verwendeten anorganisch und organischen Basen. Pharmakologisch unverträgliche Salze, die beispielsweise bei der Herstellung der erfindungsgemäßen Verbindungen im industriellen Maßstab als Verfahrensprodukte zunächst anfallen können, werden durch dem Fachmann bekannte Verfahren in pharmakologisch verträgliche Salze übergeführt. Als solche eignen sich beispielsweise wasserlösliche und wasserunlösliche Salze mit Basen, wobei als Kationen für die Salzbildung vor allem die Kationen der Alkalimetalle oder Erdalkalimetalle verwendet werden; es kommen jedoch auch die entsprechenden Kationen organischer Stickstoffbasen, wie Amine oder Aminoalkanole, Aminozucker etc. zur Anwendung. Beispielsweise seien die Salze von Natrium, Magnesium, Calcium, Dimethylamin, Diethylamin, Ethanolamin, Diethanolamin, Triethanolamin, Glucamin, N-Methylglucamin (Meglumin), Glucosamin, N-Methylglucosamin genannt.

Eine Ausgestaltung (Ausgestaltung a) der Erfindung sind 6-Aryl-3[2H]pyridazinone der oben angegebenen allgemeinen Formel I, worin
R1 Methoxy, Difluormethoxy oder Ethoxy und
R2 C4-C6-Cycloalkoxy oder C3-C6-Cycloalkylmethoxy bedeutet,
und ihre Salze mit Basen.

Eine weitere Ausgestaltung (Ausgestaltung b) der Erfindung sind 6-Aryl-3[2H]pyridazinone der oben angegebenen allgemeinen Formel I, worin
R1 C4-C6-Cycloalkoxy oder C3-C6-Cycloalkylmethoxy und
R2 Methoxy, Difluormethoxy oder Ethoxy bedeutet,
und ihre Salze mit Basen.

Die Ausgestaltung b ist gegenüber der Ausgestaltung a bevorzugt.

Bevorzugte erfindungsgemäße Verbindungen sind solche der Formel I, worin einer der Substituenten R1 und R2 Cyclopentyloxy, Cycloheptyloxy, Cyclopropylmethoxy oder Cyclobutylmethoxy, und der andere Methoxy oder Difluormethoxy bedeutet, und ihre pharmakologisch verträglichen Salze mit Basen.

Ein weiterer Gegenstand der Erfindung ist die Verwendung der erfindungsgemäßen Verbindungen bei der Behandlung oder Prophylaxe von Krankheiten, die auf der Erkrankung der Bronchien beruhen.

Ein weiterer Gegenstand der Erfindung ist die Verwendung der erfindungsgemäßen Verbindungen und ihrer pharmakologisch verträglichen Salze zur Herstellung von Arzneimitteln zur Behandlung und/oder Prophylaxe von Erkrankungen der Bronchien.

Gegenstand der Erfindung ist ferner ein Verfahren zur Herstellung der 6-Aryl-3[2H]pyridazinone der allgemeinen Formel I und ihrer Salze mit Basen, das dadurch gekennzeichnet ist, daß man
a) ein 6-Aryl-tetrahydropyridazinon der allgemeinen Formel II 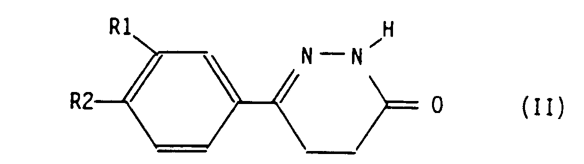 worin R1 und R2 die oben angegebene Bedeutung haben, oxydiert und gewünschtenfalls anschließend das erhaltene Pyridazinon in ein Salz überführt, oder daß man
b) eine Morpholinobuttersäure der allgemeinen Formel III 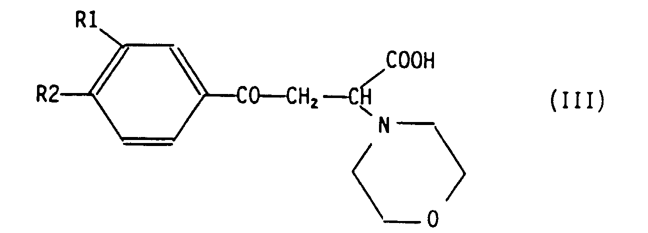 worin R1 und R2 die oben angegebene Bedeutung haben, mit Hydrazin umsetzt und gewünschtenfalls anschließend das erhaltene Pyridazinon in ein Salz überführt, oder daß man
c) eine Acrylsäure der allgemeinen Formel IV 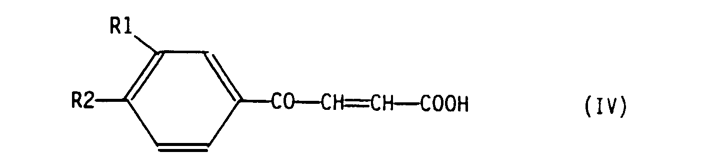 worin R1 und R2 die oben angegebene Bedeutung haben, mit Hydrazin umsetzt und gewünschtenfalls anschließend das erhaltene Pyridazinon in ein Salz überführt.

Die Durchführung des Verfahrens gemäß Variante a), b) oder c) erfolgt auf eine Weise, wie sie z.B. im europäischen Patent 163 965 beschrieben ist. Bevorzugt wird das Verfahren gemäß Variante c) durchgeführt.

Die Ausgangsverbindungen der Formel II, III und IV sind bekannt oder sie können nach bekannten Verfahren hergestellt werden, wie sie z.B. im Europäischen Patent 163 965 beschrieben sind.

Die folgenden Beispiele dienen zur näheren Erläuterung der Erfindung. Schmp. bedeutet Schmelzpunkt, h steht für Stunde(n).

### Beispiele

### 6-(3-Cyclopropylmethoxy-4-difluormethoxyphenyl)-3[2H]pyridazinon

22 g 3-Cyclopropylmethoxy-4-difluormethoxy-acetophenon werden bei Raumtemperatur zu einer Lösung von 15,8 g Glyoxylsäuremonohydrat in 15 ml Dioxan gegeben. Unter kräftigem Rühren und Stickstoffbegasung wird die Mischung für 3 h auf 100°C erhitzt und anschließend im Eisbad abgekühlt. Unter Eiskühlung werden 13 ml konz. wäßrige Ammoniaklösung so zugetropft, daß die Innentemperatur 40°C nicht übersteigt. Nach Zugabe von 8,6 g Hydrazinhydrat zur in situ gebildeten Acrylsäure wird für 1 h auf 80°C erhitzt. Nach dem Abkühlen und Zugabe von 200 ml Wasser und 200 ml gesättigter Ammoniumchlorid-Lösung wird dreimal mit Essigester extrahiert. Die vereinigten organischen Phasen werden über Magnesiumsulfat getrocknet und eingeengt. Das erhaltene Rohprodukt wird über eine Kieselgelsäule (Laufmittel: Acetonitril/Ammoniak 9:1) gereinigt und aus Essigester umkristallisiert. Man erhält 18,3 g (69 %) der Titelverbindung vom Schmp. 141,5°C.

Auf analoge Weise erhält man die folgenden Pyridazinone:
6-(4-Cyclopropylmethoxy-3-difluormethoxyphenyl)-3[2H]pyridazinon, Schmp. 172°C
6-(3-Cyclopentyloxy-4-methoxyphenyl)-3[2H]pyridazinon, Schmp. 201°C
6-(3-Cycloheptyloxy-4-methoxyphenyl)-3[2H]pyridazinon, Schmp. 172°C
6-(3-Cyclobutylmethoxy-4-methoxyphenyl)-3[2H]pyridazinon, Schmp. 201°C
6-(3-Cycloheptyloxy-4-difluormethoxyphenyl)-3[2H]pyridazinon, Schmp. 139-140°C
6-(3-Cyclopentyloxy-4-difluormethoxyphenyl)-3[2H]pyridazinon, Schmp. 113°C

### Gewerbliche Anwendbarkeit

Die erfindungsgemäßen 6-Aryl-3[2H]pyridazinone besitzen wertvolle pharmakologische Eigenschaften, die sie gewerblich verwertbar machen. Sie zeichnen sich vor allem durch solche Eigenschaften aus, die sie für die Therapie von Atemwegserkrankungen verschiedener Genese geeignet erscheinen lassen. Insbesondere können entzündliche und allergeninduzierte Bronchialerkrankungen aufgrund der antiinflammatorischen und broncholytischen Wirksamkeit der erfindungsgemäßen Verbindungen behandelt werden, wobei die vergleichsweise überraschend starke antientzündliche Wirkung der erfindungsgemäßen Verbindungen besonders hervorgehoben werden soll. Daneben zeichnen sich die erfindungsgemäßen Verbindungen durch eine geringe Toxizität, eine große therapeutische Breite und das Fehlen wesentlicher Nebenwirkungen aus.

Die broncholytische und antiinflammatorische Wirksamkeit der 6-Aryl-3[2H]pyridazinone ermöglicht ihren Einsatz in der Human- und Veterinärmedizin, wobei sie zur Behandlung und Prophylaxe von Krankheiten, die auf Erkrankungen der Bronchien beruhen, verwendet werden. Beispielsweise können akute und chronisch obstruktive Atemwegserkrankungen verschiedener Genese (Bronchitis, allergische Bronchitis, Asthma bronchiale) bei Mensch und Tier behandelt werden.

Weiterer Gegenstand der Erfindung sind die erfindungsgemäßen Verbindungen zur Anwendung bei der Behandlung und/oder Prophylaxe von Krankheiten, die auf Erkrankungen der Bronchien beruhen.

Ebenso betrifft die Erfindung die Verwendung der erfindungsgemäßen Verbindungen zur Herstellung von Arzneimitteln, die zur Behandlung und/oder Prophylaxe von Krankheiten, die auf Erkrankungen der Bronchien beruhen, eingesetzt werden.

Weiterhin sind Arzneimittel zur Behandlung und/oder Prophylaxe von Krankheiten, die auf Erkrankungen der Bronchien beruhen und die eine oder mehrere der erfindungsgemäßen Verbindungen und/oder ihre pharmakologisch verträglichen Salze enthalten, Gegenstand der Erfindung.

Die erfindungsgemäßen Arzneimittel werden nach an sich bekannten Verfahren hergestellt, wobei bezüglich der Zubereitungen, Dosierungen, Darreichungsformen etc. beispielsweise auf die Ausführungen im Europäischen Patent 163 965 verwiesen wird.

## Patentansprüche (Patentansprüche für folgende(n) Vertragsstaat(en): AT, BE, CH, DE, DK, FR, GB, IT, LI, LU, NL, SE)

1. 6-Aryl-3[2H]pyridazinone der allgemeinen Formel I 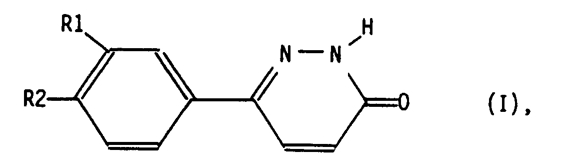 worin einer der Substituenten R1 und R2 Methoxy, Difluormethoxy oder Ethoxy, und der andere C4-C7-Cycloalkoxy oder C3-C7-Cycloalkylmethoxy bedeutet, und ihre Salze mit Basen.

2. Verbindungen der Formel I nach Anspruch 1, worin
R1 Methoxy, Difluormethoxy oder Ethoxy und
R2 C4-C6-Cycloalkoxy oder C3-C6-Cycloalkylmethoxy bedeutet,
und ihre Salze mit Basen.

3. Verbindungen der Formel I nach Anspruch 1, worin
R1 C4-C6-Cycloalkoxy oder C3-C6-Cycloalkylmethoxy und
R2 Methoxy, Difluormethoxy oder Ethoxy bedeutet,
und ihre Salze mit Basen.

4. Verbindungen der Formel I nach Anspruch 1, worin einer der Substituenten R1 und R2 Cyclopentyloxy, Cycloheptyloxy, Cyclopropylmethoxy oder Cyclobutylmethoxy und der andere Methoxy oder Difluormethoxy bedeutet, und ihre pharmakologisch verträglichen Salze mit Basen.

5. Verfahren zur Herstellung der 6-Aryl-3[2H]pyridazinone der allgemeinen Formel I nach Anspruch 1 und ihrer Salze mit Basen, dadurch gekennzeichnet, daß man
a) ein 6-Aryl-tetrahydropyridazinon der allgemeinen Formel II 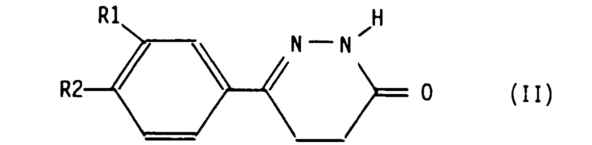 worin R1 und R2 die in Anspruch 1 angegebene Bedeutung haben, oxydiert und gewünschtenfalls anschließend das erhaltene Pyridazinon in ein Salz überführt, oder daß man
b) eine Morpholinobuttersäure der allgemeinen Formel III 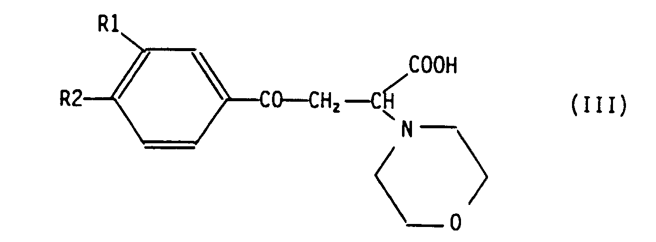 worin R1 und R2 die in Anspruch 1 angegebene Bedeutung haben, mit Hydrazin umsetzt und gewünschtenfalls anschließend das erhaltene Pyridazinon in ein Salz überführt, oder daß man
c) eine Acrylsäure der allgemeinen Formel IV 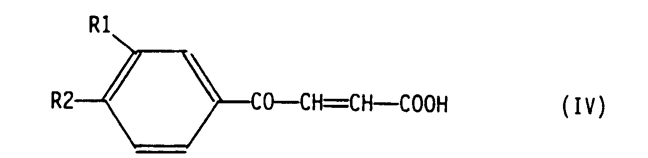 worin R1 und R2 die in Anspruch 1 angegebene Bedeutung haben, mit Hydrazin umsetzt und gewünschtenfalls anschließend das erhaltene Pyridazinon in ein Salz überführt.

6. Arzneimittel enthaltend eine oder mehrere Verbindungen nach einem der Ansprüche 1, 2, 3 oder 4 und/oder ihre pharmakologisch verträglichen Salze.

7. Verbindungen nach einem der Ansprüche 1, 2, 3 oder 4 und/oder ihre pharmakologisch verträglichen Salze zur Anwendung bei der Behandlung und/oder Prophylaxe von Erkrankungen der Bronchien.

8. Verwendung der Verbindungen nach einem der Ansprüche 1, 2, 3 oder 4 und/oder ihrer pharmakologisch verträglichen Salze zur Herstellung von Arzneimitteln zur Behandlung und/oder Prophylaxe von Erkrankungen der Bronchien.

## Patentansprüche (Patentansprüche für folgende(n) Vertragsstaat(en): ES, GR)

1. Verfahren zur Herstellung der 6-Aryl-3[2H]pyridazinone der allgemeinen Formel I 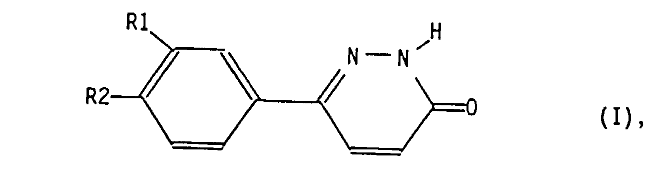 worin einer der Substituenten R1 und R2 Methoxy, Difluormethoxy oder Ethoxy, und der andere C4-C7-Cycloalkoxy oder C3-C7-Cycloalkylmethoxy bedeutet, und ihrer Salze mit Basen, dadurch gekennzeichnet, daß
a) ein 6-Aryl-tetrahydropyridazinon der allgemeinen Formel II 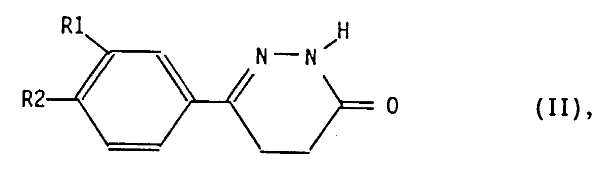 worin R1 und R2 die oben angegebene Bedeutung haben, oxydiert und gewünschtenfalls anschließend das erhaltene Pyridazinon in ein Salz überführt, oder daß man
b) eine Morpholinobuttersäure der allgemeinen Formel III 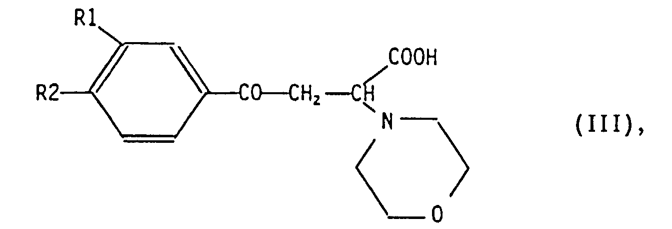 worin R1 und R2 die oben angegebene Bedeutung haben, mit Hydrazin umsetzt und gewünschtenfalls anschließend das erhaltene Pyridazinon in ein Salz überführt, oder daß man
c) eine Acrylsäure der allgmeinen Formel IV 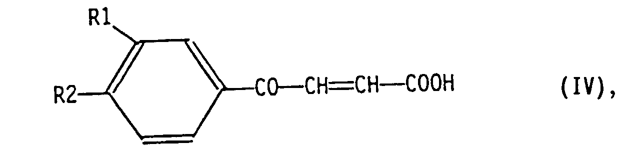 worin R1 und R2 die oben angegebene Bedeutung haben, mit Hydrazin umsetzt und gewünschtenfalls anschließend das erhaltene Pyridazinon in ein Salz überführt.

2. Verfahren nach Anspruch 1 zur Herstellung der Verbindungen der Formel I nach Anspruch 1, worin
R1 Methoxy, Difluormethoxy oder Ethoxy und
R2 C4-C6-Cycloalkoxy oder C3-C6-Cycloalkylmethoxy bedeutet, und ihrer Salze.

3. Verfahren nach Anspruch 1 zur Herstellung der Verbindungen der Formel I nach Anspruch 1, worin
R1 C4-C6-Cycloalkoxy oder C3-C6-Cycloalkylmethoxy und
R2 Methoxy, Difluormethoxy oder Ethoxy bedeutet, und ihrer Salze mit Basen.

4. Verfahren nach Anspruch 1 zur Herstellung der Verbindungen der Formel I nach Anspruch 1, worin einer der Substituenten R1 und R2 Cyclopentyloxy, Cycloheptyloxy, Cyclopropylmethoxy oder Cyclobutylmethoxy und der andere Methoxy oder Difluormethoxy bedeutet, und ihrer pharmakologisch verträglichen Salze mit Basen.

5. Verbindungen nach einem der Ansprüche 1, 2, 3 oder 4 und/oder ihre pharmakologisch verträglichen Salze zur Anwendung bei der Behandlung und/oder Prophylaxe von Erkrankungen der Bronchien.

6. Verwendung der Verbindungen nach einem der Ansprüche 1, 2, 3 oder 4 und/oder ihrer pharmakologisch verträglichen Salze zur Herstellung von Arnzeimmitteln zur Behandlung und/oder Prophylaxe von Erkrankungen der Bronchien.

## Claims (Claims for the following Contracting State(s): AT, BE, CH, LI, DE, DK, FR, GB, IT, LU, NL, SE)

1. 6-Aryl-3[2H]pyridazinones of the general formula I 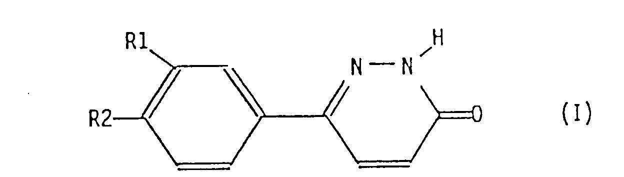 wherein one of the substituents R1 and R2 denotes methoxy, difluoromethoxy or ethoxy and the other denotes C4-C7-cycloalkoxy or C3-C7-cycloalkylmethoxy, and their salts with bases.

2. Compounds of formula I according to claim 1, wherein
R1 denotes methoxy, difluoromethoxy or ethoxy,
R2 denotes C4-C6-cydoalkoxy or C3-C6-cycloalkylmethoxy,
and their salts with bases.

3. Compounds of formula I according to claim 1, wherein
R1 denotes C4-C6-cydoalkoxy or C3-C6-cycloalkylmethoxy and
R2 denotes methoxy, difluoromethoxy or ethoxy,
and their salts with bases.

4. Compounds of formula I according to claim 1, wherein one of the substituents R1 and R2 denotes cyclopentyloxy, cycloheptyloxy, cyclopropylmethoxy or cyclobutylmethoxy, and the other denotes methoxy or difluoromethoxy, and their pharmacologically tolerated salts with bases.

5. Process for the preparation of the 6-aryl-3[2H]pyridazinones of the general formula I according to claim 1 and of their salts with bases, which comprises
a) oxidizing a 6-aryltetrahydropyridazinone of the general formula II 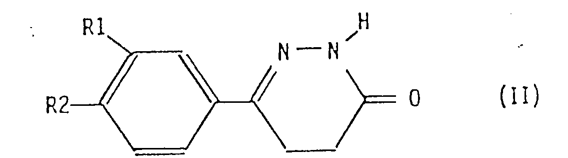 in which R1 and R2 have the meaning given in claim 1, and, if desired, then converting the resulting pyridazinone into a salt, or
b) reacting a morpholinobutyric acid of the general formula III 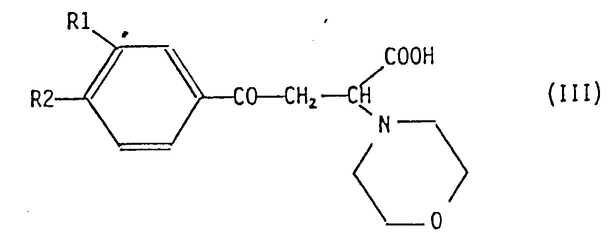 in which R1 and R2 have the meaning given in claim 1, with hydrazine and, if desired, then converting the resulting pyridazinone into a salt, or
c) reacting an acrylic acid of the general formula IV 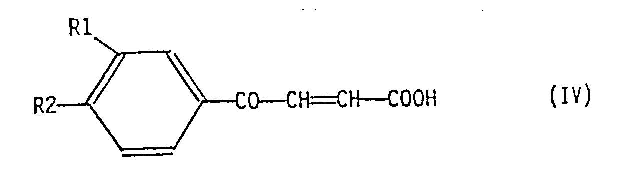 in which R1 and R2 have the meaning given in claim 1, with hydrazine and, if desired, then converting the resulting pyridazinone into a salt.

6. Medicaments containing one or more compounds according to one of claims 1, 2, 3 or 4 and/or their pharmacologically tolerated salts.

7. Compounds according to one of claims 1, 2, 3 or 4 and/or their pharmacologically tolerated salts for use in the treatment and/or prophylaxis of diseases of the bronchi.

8. Use of the compounds according to one of claims 1, 2, 3 or 4 and/or their pharmacologically tolerated salts for the preparation of medicaments for the treatment and/or prophylaxis of diseases of the bronchi.

## Claims (Claims for the following Contracting State(s): ES, GR)

1. Process for the preparation of the 6-aryl-3[2H]pyridazinones of the general formula I 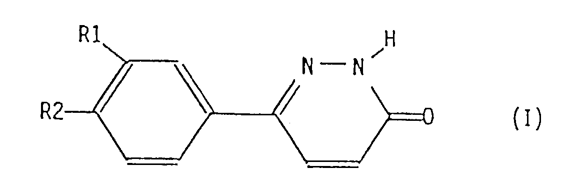 wherein one of the substituents R1 and R2 denotes methoxy, difluoromethoxy or ethoxy and the other denotes C4-C7-cycloalkoxy or C3-C7-cycloalkylmethoxy, and of their salts with bases, which comprises
a) oxidizing a 6-aryltetrahydropyridazinone of the general formula II 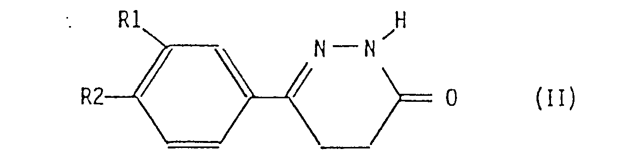 in which R1 and R2 have the meaning given above, and, if desired, then converting the resulting pyridazinone into a salt, or
b) reacting a morpholinobutyric acid of the general formula III 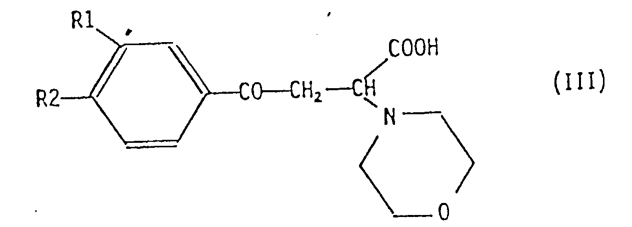 in which R1 and R2 have the meaning given above, with hydrazine and, if desired, then converting the resulting pyridazinone into a salt, or
c) reacting an acrylic acid of the general formula IV 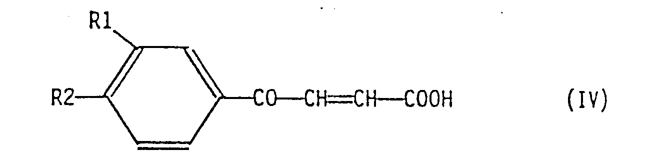 in which R1 and R2 have the meaning given above, with hydrazine and, if desired, then converting the resulting pyridazinone into a salt.

2. Process according to claim 1 for the preparation of compounds of formula I according to claim 1, wherein
R1 denotes methoxy, difluoromethoxy or ethoxy,
R2 denotes C4-C6-cydoalkoxy or C3-C6-cycloalkylmethoxy,
and of their salts with bases.

3. Process according to claim 1 for the preparation of compounds of formula I according to claim 1, wherein
R1 denotes C4-C6-cydoalkoxy or C3-C6-cycloalkylmethoxy and
R2 denotes methoxy, difluoromethoxy or ethoxy,
and of their salts with bases.

4. Process according to claim 1 for the preparation of compounds of formula I according to claim 1, wherein one of the substituents R1 and R2 denotes cyclopentyloxy, cycloheptyloxy, cyclopropylmethoxy or cyclobutylmethoxy, and the other denotes methoxy or difluoromethoxy, and of their pharmacologically tolerated salts with bases.

5. Compounds according to one of claims 1, 2, 3 or 4 and/or their pharmacologically tolerated salts for use in the treatment and/or prophylaxis of diseases of the bronchi.

6. Use of the compounds according to one of claims 1, 2, 3 or 4 and/or their pharmacologically tolerated salts for the preparation of medicaments for the treatment and/or prophylaxis of diseases of the bronchi.

## Revendications (Revendications pour l'(les) Etat(s) contractant(s) suivant(s): AT, BE, CH, DE, DR, FR, GB, IT, LI, LU, NL, SE)

1. 6-aryl-3[2H]pyridazinones de formule générale (I) 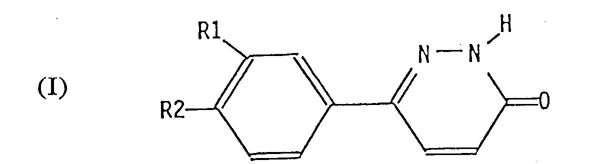 dans laquelle un des substituants R₁ et R₂ représente un groupe méthoxy, difluorométhoxy ou éthoxy, et l'autre un groupe cycloalcoxy en C₄₋₇ ou (cycloalkyle en C₃₋₇)-méthoxy, et les sels d'addition de bases de tels composés.

2. Composés de formule (I) selon la revendication 1, dans lesquels R₁ représente un groupe méthoxy, difluorométhoxy ou éthoxy et R₂ représente un groupe cycloalcoxy en C₄₋₆ ou (cycloalkyle en C₃₋₆)-méthoxy, et les sels d'addition de bases de tels composés.

3. Composés de formule (I) selon la revendication 1, dans lesquels R₁ représente un groupe cycloalcoxy en C₄₋₆ ou (cycloalkyle en C₃₋₆)-méthoxy, et R₂ représente un groupe méthoxy, difluorométhoxy ou éthoxy, et les sels d'addition de bases de tels composés.

4. Composés de formule (I) selon la revendication 1, dans lesquels un des substituants R₁ et R₂ représente un groupe cyclopentyloxy, cycloheptyloxy, cyclopropylméthoxy ou cyclobutylméthoxy et l'autre substituant est un groupe méthoxy ou difluorométhoxy, et les sels d'addition de bases pharmaceutiquement acceptables de tels composés.

5. Procédé de préparation de 6-aryl-3[2H]pyridazinones de formule générale (I) selon la revendication 1 et des sels d'addition de bases de ces composés, caractérisé
a) en ce que l'on soumet une 6-aryl-tétrahydropyridazinone de formule générale (II) 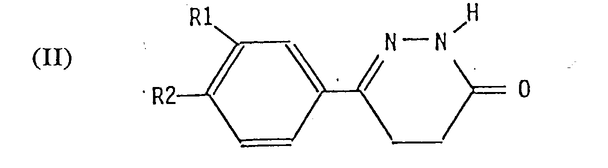 dans laquelle R₁ et R₂ ont la signification indiquée dans la revendication 1, à une oxydation, et en ce que l'on convertit éventuellement la pyridazinone obtenue en un sel, ou
b) en ce que l'on fait réagir l'acide morpholinobutyrique de formule générale (III) 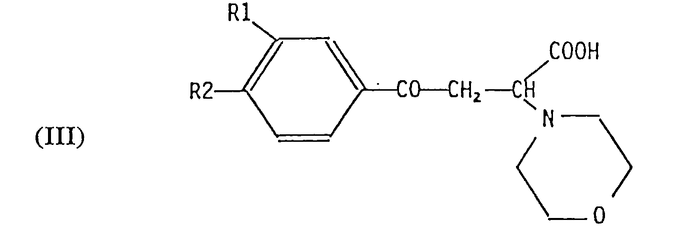 dans laquelle R₁ et R₂ ont la signification indiquée dans la revendication 1, avec de l'hydrazine et en ce que l'on convertit ensuite éventuellement la pyridazinone obtenue en un sel, ou
c) en ce que l'on fait réagir un acide acrylique de formule générale (IV) 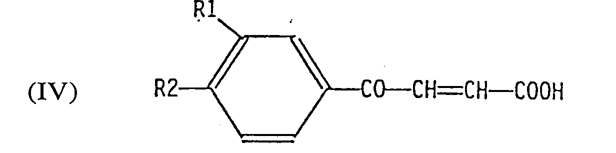 dans laquelle R₁ et R₂ ont la signification indiquée dans la revendication 1, avec de l'hydrazine et en ce que l'on convertit ensuite éventuellement la pyridazinone obtenue en un sel.

6. Médicament contenant un ou plusieurs composés selon une des revendications 1, 2, 3 ou 4 et/ou les sels pharmaceutiquement acceptables de ceux-ci.

7. Composés selon une des revendications 1, 2, 3 ou 4 et/ou les sels pharmaceutiquement acceptables de ces composés pour l'utilisation dans le traitement thérapeutique et/ou prophylactique de maladies des bronches.

8. Utilisation des composés selon l'une des revendications 1, 2, 3 ou 4 et/ou des sels pharmaceutiquement acceptables de tels composés pour la préparation de médicaments destinés au traitement thérapeutique et/ou prophylactique de maladies des bronches.

## Revendications (Revendications pour l'(les) Etat(s) contractant(s) suivant(s): ES, GR)

1. Procédé de préparation de 6-aryl-3[2H]pyridazinones de formule générale (I) 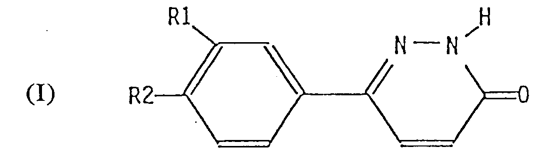 dans laquelle un des substituants R₁ et R₂ représente un groupe méthoxy, difluorométhoxy ou éthoxy, et l'autre un groupe cycloalcoxy en C₄₋₇ ou (cycloalkyle en C₃₋₇)-méthoxy, et les sels d'addition de bases de tels composés, caractérisé
a) en ce que l'on soumet une 6-aryl-tétrahydropyridazinone de formule générale (II) 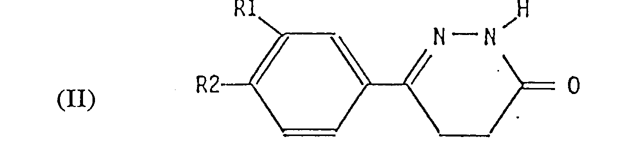 dans laquelle R₁ et R₂ ont la signification indiquée ci-dessus, à une oxydation, et en ce que l'on convertit éventuellement la pyridazinone obtenue en un sel, ou
b) en ce que l'on fait réagir un acide morpholinobutyrique de formule générale (III) 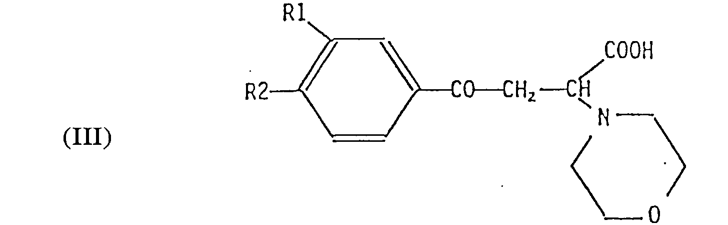 dans laquelle R₁ et R₂ ont la signification indiquée ci-dessus, avec de l'hydrazine et en ce que l'on convertit éventuellement la pyridazinone obtenue en un sel, ou
c) en ce que l'on fait réagir un acide acrylique de formule générale (IV) 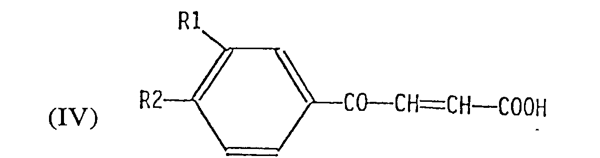 dans laquelle R₁ et R₂ ont la signification indiquée ci-dessus, avec de l'hydrazine et en ce que l'on convertit ensuite éventuellement la pyridazinone obtenue en un sel.

2. Procédé selon la revendication 1 pour la préparation de composés de formule (I), dans lesquels R₁ représente un groupe méthoxy, difluorométhoxy ou éthoxy et R₂ représente un groupe cycloalcoxy en C₄₋₆ ou (cycloalkyle en C₃₋₆)-méthoxy, et les sels d'addition de bases de tels composés.

3. Procédé selon la revendication 1 pour la préparation de composés, dans lesquels R₁ représente un groupe cycloalcoxy en C₄₋₆ ou (cycloalkyle en C₃₋₆)-méthoxy, et R₂ représente un groupe méthoxy, difluorométhoxy ou éthoxy, et les sels d'addition de bases de tels composés.

4. Procédé selon la revendication 1 pour la préparation de composés de formule (I) selon la revendication 1, dans lesquels un des substituants R₁ et R₂ représente un groupe cyclopentyloxy, cycloheptyloxy, cyclopropylméthoxy ou cyclobutylméthoxy et l'autre substituant est un groupe méthoxy ou difluorométhoxy, et de sels d'addition de bases pharmaceutiquement acceptables de ces composés.

5. Composés selon une des revendications 1, 2, 3 ou 4 et/ou les sels pharmaceutiquement acceptables de ces composés pour l'utilisation dans le traitement thérapeutique et/ou prophylactique de maladies des bronches.

6. Utilisation des composés selon l'une des revendications 1, 2, 3 ou 4 et/ou des sels pharmaceutiquement acceptables de tels composés pour la préparation de médicaments destinés au traitement thérapeutique et/ou prophylactique de maladies des bronches.
